# EUROPÄISCHE PATENTSCHRIFT

(11) **EP 4 633 546 B1**
(45) Veröffentlichungstag und Bekanntmachung des Hinweises auf die Patenterteilung: **22.07.2026**
(21) Anmeldenummer: 23833384.3
(22) Anmeldetag: 15.12.2023
(51) Int. Cl.: A61F 2/66, A61F 2/74, A61F 2/68

(54) **PROTHESENFUSS**
PROSTHETIC FOOT
PIED PROTHÉTIQUE

(30) Priorität: 16.12.2022 DE 102022133618
(43) Veröffentlichungstag der Anmeldung: 22.10.2025
(73) Patentinhaber: Ottobock SE & Co. KGaA, 37115 Duderstadt (DE)
(72) Erfinder: PUSCH, Martin, 37115 Duderstadt (DE); SCHUH, Andreas, 37115 Duderstadt (DE); WELLING, Malte, 37115 Duderstadt (DE)
(74) Vertreter: Gramm, Lins & Partner Patent- und Rechtsanwälte PartGmbB
(86) Internationale Anmeldenummer: PCT/EP2023/086043
(87) Internationale Veröffentlichungsnummer: WO 2024/126783

(56) Entgegenhaltungen:
- EP-B1- 1 933 775
- US-A1- 2017 042 703

## Beschreibung

Die Erfindung betrifft einen Prothesenfuß mit einem Fußelement, einem proximalen Anschlusselement, das an dem Fußelement angeordnet ist, und einer Sperreinrichtung, die in eine Freigabestellung und in eine Sperrstellung bringbar ist, wobei eine Orientierung, in der das Anschlusselement an dem Fußelement angeordnet ist, veränderbar ist, wenn sich die Sperreinrichtung in der Freigabestellung befindet.

Derartige Prothesenfüße sind aus dem Stand der Technik bekannt. Das proximale Anschlusselement dient dazu, den Prothesenfuß an einem anderen Prothesenelement, beispielsweise einem Unterschenkelrohr zu befestigen. An dem Anschlusselement ist das Fußelement angeordnet. Die Orientierung zwischen dem Anschlusselement und dem Fußelement ist veränderbar, wenn sich die Sperreinrichtung in der Freigabestellung befindet. Dadurch kann beispielsweise auf eine sich verändernde Absatzhöhe reagiert werden, wenn der Träger des Prothesenfußes einen Schuh, in den der Prothesenfuß eingeführt wird, wechselt. **In** diesem Fall wird die Sperreinrichtung aus der Sperrstellung in die Freigabestellung gebracht. Dies geschieht in der Regel, indem ein mechanisches Betätigungselement manuell betätigt wird. Dieses Betätigungselement ist beispielswiese ein Druckknopf oder ein ähnliches Element, durch dessen Betätigung zwei Bauteile der Sperreinrichtung außer Eingriff gebracht werden, wodurch die Sperreinrichtung in die Freigabestellung gebracht wird. Dann kann die Orientierung zwischen Fußelement und proximalem Anschlusselement verändert werden. Bevorzugt bedeutet das, dass das Fußelement relativ zu dem proximalen Anschlusselement um eine Schwenkachse verschwenkt wird. Diese Schwenkachse verläuft vorzugsweise in medial-laterale Richtung, also quer zur Längserstreckung des Fußes, die sich von der Ferse zu den Zehen erstreckt.

Ist die gewünschte Orientierung gefunden, wird die Sperreinrichtung aus der Freigabestellung in die Sperrstellung gebracht. **In** der Sperrstellung verhindert die Sperreinrichtung, dass die Orientierung zwischen dem Fußelement und dem proximalen Anschlusselement verändert wird. **In** vielen Ausgestaltungen wird dazu das Betätigungselement, das bereits betätigt wurde, um die Sperreinrichtung aus der Sperrstellung in die Freigabestellung zu bringen, erneut betätigt. Wird das Betätigungselement betätigt, wenn sich die Sperreinrichtung in der Freigabestellung befindet, wird dadurch die Sperreinrichtung in die Sperrstellung gebracht. Dies geschieht beispielsweise dadurch, dass die beiden Bauteile der Sperreinrichtung wieder in Eingriff gebracht werden.

Nachteilig ist, dass das Betätigungselement betätigt werden muss, um die Sperreinrichtung aus der Freigabestellung in die Sperrstellung zu bringen. Da sich das Betätigungselement in der Regel an dem Fußelement oder an dem proximalen Anschlusselement befindet, muss sich der Träger des Prothesenfußes bewegen, beispielsweise bücken, um dieses Betätigungselement betätigen zu können. Dies ist wenig problematisch, um die Sperreinrichtung aus der Sperrstellung in die Freigabestellung zu bringen, weil sich die Sperreinrichtung zu diesem Zeitpunkt in der Sperrstellung befindet und daher die Orientierung zwischen dem Fußelement und dem Anschlusselement nicht veränderbar ist. Wenn die Sperreinrichtung jedoch aus der Freigabestellung in die Sperrstellung gebracht werden soll, befindet sie sich in der Freigabestellung, so dass eine Orientierung zwischen dem Fußelement und dem proximalen Anschlusselement veränderbar ist. Durch die benötigte Bewegung zum Betätigen des Betätigungselementes kann eine solche Veränderung der Orientierung leicht versehentlich geschehen, so dass die gerade gefundene gewünschte Orientierung wieder verändert wird. EP 1 933 775 B1 beschreibt einen Prothesenfuß mit den Merkmalen des Oberbegriffs des Anspruchs 1.

Der Erfindung liegt daher die Aufgabe zugrunde, einen Prothesenfuß derart zu verbessern, dass die Orientierung weniger leicht verändert werden kann, wenn die Veränderbarkeit wieder beendet werden soll.

Die Erfindung löst die gestellte Aufgabe durch einen Prothesenfuß gemäß dem Oberbegriff des Anspruchs 1, der sich dadurch auszeichnet, dass die Sperreinrichtung derart ausgebildet ist, dass die Sperreinrichtung selbsttätig aus der Freigabestellung in die Sperrstellung gelangt, nachdem ein Schaltmoment auf das Fußelement aufgebracht wird.

Das Schaltmoment, das beispielsweise ein Kraftstoß oder ein Drehmoment sein kann, sorgt also dafür, dass die Sperreinrichtung aus der Freigabestellung in die Sperrstellung gelangt, wobei kein elektronisches Bauteil, also beispielsweise kein Sensor und keine elektrische Steuerung, beispielsweise in Form einer elektronischen Datenverarbeitungseinrichtung, verwendet wird. Das Schaltmoment wird nicht durch einen Sensor detektiert und dann das elektronische Sensorsignal dazu verwendet, die Sperreinrichtung zu schalten, also von der Freigabestellung in die Sperrstellung zu bringen.

Da bei einem erfindungsgemäßen Prothesenfuß ein Schaltmoment auf das Fußelement aufgebracht werden muss, um die Sperreinrichtung aus der Freigabestellung in die Sperrstellung zu bringen, kann darauf verzichtet werden, dass zusätzlich ein Betätigungselement betätigt werden muss. Dies ist zwar möglich, jedoch nicht bevorzugt. Bevorzugt kann das Schaltmoment auf das Fußelement aufgebracht werden, wenn der Träger des Prothesenfußes aufrecht steht. Eine Bewegung des Trägers, um mit einer Hand ein Betätigungselement zu erreichen, das gegebenenfalls am Fußelement oder am proximalen Anschlusselement angeordnet ist, kann dann entfallen. Damit entfällt das Risiko, bei einer solchen Bewegung die gefundene und für gut befundene Orientierung zwischen dem Fußelement und proximalen Anschlusselement wieder zu verändern.

Bei der Orientierung des Fußelementes relativ zu dem Anschlusselement handelt es sich um die Orientierung, in der sich die beiden Elemente zueinander ohne die Einwirkung äußerer Kräfte befinden. Man könnte von einer Null-Stellung oder einer Ruhe-Orientierung sprechen. In bevorzugten Ausgestaltungen kann das Fußelement nicht relativ zu dem Anschlusselement bewegt werden, wenn sich die Sperreinrichtung in der Sperrstellung befindet. **In** alternativen Ausgestaltungen ist es jedoch möglich, das Fußelement relativ zu dem Anschlusselement zu bewegen, insbesondere um die Schwenkachse zu verschwenken, selbst wenn sich die Sperreinrichtung in der Sperrstellung befindet. Dies ist jedoch keine Veränderung der Orientierung im Sinne der vorliegenden Erfindung, sondern lediglich eine Veränderung der momentanen Position der beiden Elemente relativ zueinander. Diese momentane Position lässt sich innerhalb eines vorbestimmten Bereichs, beispielsweise eines vorgegebenen Winkelbereichs, verändern. Dazu muss eine äußere Kraft auf das Fußelement oder das Anschlusselement wirken, die zu der Bewegung der beiden Elemente relativ zueinander führt. Die Orientierung, also die Null-Stellung oder Ruhe-Orientierung, die ohne die äußeren Kräfte vorliegt, wird dadurch nicht beeinflusst.

Das Schaltmoment bringt die Sperreinrichtung aus der Freigabestellung in die Sperrstellung. Das bedeutet jedoch nicht zwangsläufig, dass das Schaltmoment aufgebracht werden muss, wenn sich die Sperreinrichtung in der Fraigabestellung befinden. **In** besonderen Ausführungsformen der vorliegenden Erfindung wird das Schaltmoment aufgebracht, wenn sich die Sperreinrichtung in der Sperrstellung befindet. Sie wird bevorzugt durch das Schaltmoment in die Freigabestellung gebracht und gelangt von dort selbsttätig, also ohne, dass ein weiteres Moment aufgebracht oder eine Betätigungselement betätigt werden muss, wieder in die Sperrstellung zurück. Bevorzugt ist in diesen Ausführungsformen ein Verzögerungselement vorhanden, durch dass der Moment, zu dem die Sperreinrichtung aus der Freigabestellung in die Sperrstellung gelangt verzögert wird. Die Sperrstellung gelangt also in diesem Ausführungsbeispielen durch das Schaltmoment in die Freigabestellung. Dadurch wird die Orientierung zwischen Fußelement und Anschlusselement, also beispielsweise einem Unterschenkel, einstellbar. Der Zeitraum, in dem dies möglich ist, wird durch das Verzögerungselement bestimmt. Sobald die Sperreinrichtung wieder in die Sperrstellung gelangt, ist die Orientierung nicht mehr veränderbar.

Das Schaltmoment wird auf das Fußelement aufgebracht. Dies muss nicht zwangsläufig homogen oder auf alle Elemente, die das Fußelement bilden, erfolgen. Es ist ausreichend und erfindungsgemäß, wenn das Schaltmoment beispielsweise nur auf ein Bauteil des Fußelementes ausgeübt wird. Dieses Bauteil ist vorzugsweise das Betätigungselement, durch dessen Betätigung die Sperreinrichtung aus der Sperrstellung in die Freigabestellung gelangt.

Vorzugsweise ist das Fußelement relativ zu dem proximalen Anschlusselement um eine Schwenkachse schwenkbar, wobei das Schaltmoment ein Drehmoment um diese Schwenkachse ist. Ein solches Drehmoment, dessen Stärke vorzugsweise einen vorbestimmten Grenzwert überschreiten muss, um als Schaltmoment zu wirken, kann beispielsweise aufgebracht werden, indem der Träger des Prothesenfußes die Belastung des Prothesenfußes ändert. Die Schwenkachse kann sich in medial-lateraler Richtung erstrecken und die Achse sein, um die das Fußelement relativ zu dem proximalen Anschlusselement verschwenkt wird, um die Orientierung zwischen den beiden Elementen einzustellen. Um in diesem Fall das Schaltmoment aufzubringen, ist es vorzugsweise ausreichend, wenn der Träger des Prothesenfußes sein Gewicht nach vorn in Richtung auf den Vorfuß oder nach hinten in Richtung auf die Ferse verlagert.

Bevorzugt ist das Schaltmoment ein Drehmoment um eine Schwenkachse, die sich von einem Fersenbereich des Prothesenfußes zu einem Vorfußbereich des Prothesenfußes erstreckt. Das Schaltmoment kann in diesem Fall erzeugt werden, indem der Träger des Prothesenfußes den Prothesenfuß nach rechts oder links belastet. Dies hat den Vorteil, dass das Schaltmoment nicht dazu führen kann, dass die Orientierung zwischen dem Fußelement und dem proximalen Anschlusselement verändert wird. Die Sperreinrichtung ist auch in dieser Ausgestaltung eingerichtet, lediglich die Bewegung um die medial-lateral verlaufende Schwenkachse zu beeinflussen, um die die beiden Elemente relativ zueinander bewegt werden, um die Orientierung anzupassen.

Vorzugsweise weist der Prothesenfuß ein Betätigungselement, beispielsweise einen Druckknopf, auf. Durch Betätigen dieses Betätigungselementes wird vorzugsweise die Sperreinrichtung aus der Sperrstellung in die Freigabestellung gebracht. Das Schaltmoment führt bevorzugt dazu, dass das Betätigungselement erneut betätigt wird und so die Sperreinrichtung aus der Freigabestellung in die Sperrstellung zurückkehrt. Vorzugsweise weist der Prothesenfuß ein Verzögerungselement auf, das ausgebildet und eingerichtet ist, die Wirkung des Schaltmomentes zu verzögern. Dadurch wird dem Träger des Prothesenfußes Zeit gegeben, die Orientierung zwischen dem Fußelement und dem Anschlusselement wie gewünscht einzustellen, bevor die Sperreinrichtung aus der Freigabestellung in die Sperrstellung gebracht wird.

Bevorzugt weist das Verzögerungselement ein Bauteil auf, das sich in einer ersten Position befindet, wenn sich die Sperreinrichtung in der Sperrstellung befindet, und das sich in einer zweiten Position befindet, wenn die Sperreinrichtung sich in der Freigabestellung befindet. Dieses Bauteil kann beispielsweise das Betätigungselement, vorzugsweise das mechanische Betätigungselement, sein, durch dessen Betätigung die Sperreinrichtung aus der Sperrstellung in die Freigabestellung bringbar ist. Befindet sich die Sperreinrichtung in der Sperrstellung, befindet sich das Betätigungselement in der ersten Position. Aus dieser ersten Position wird das Betätigungselement vorzugsweise in die zweite Position gebracht, wodurch die Sperreinrichtung in die Freigabestellung gebracht wird. Alternativ oder zusätzlich dazu wird ein anderes Bauteil als das Betätigungselement in die zweite Position gebracht, wenn die Sperreinrichtung aus der Sperrstellung in die Freigabestellung gebracht wird.

Das Bauteil wird durch einen Haltemechanismus in der zweiten Position gehalten. Um das Bauteil wieder in die erste Position und damit die Sperreinrichtung wieder in die Sperrstellung zu bringen, wird der Haltemechanismus durch das Schaltmoment deaktiviert. Das Bauteil wird dann bevorzugt einer Kraft ausgesetzt, die es aus der zweiten Position in die erste Position bringt. Diese Kraft wird beispielsweise durch eine Feder aufgebracht.

Vorzugsweise wird ein Fluid, beispielsweise eine Flüssigkeit oder ein Gas, vorzugsweise Luft, aus einem ersten Volumen herausgedrückt oder in das erste Volumen hineingesogen, wenn das Bauteil aus der zweiten Position in die erste Position gebracht wird. Dazu wird das Fluid vorzugsweise durch einen Kanal oder eine Leitung bewegt, die durch ihre Geometrie einen Strömungswiderstand aufweist und diesen dem Fluid entgegensetzt. Je größer dieser Strömungswiderstand ist, desto weniger Fluid wird bei gleicher Kraft durch den Kanal oder die Leitung bewegt. Die Menge des durch den Kanal oder die Leitung strömenden Fluids begrenzt vorzugsweise die Geschwindigkeit, mit der die Kraft das Bauteil aus der zweiten Position in die erste Position und damit die Sperreinrichtung aus der Freigabestellung in die Sperrstellung bringen kann.

Vorzugsweise weist der Kanal oder die Leitung ein Ventil auf, durch das der Strömungswiderstand einstellbar ist, vorzugsweise, indem der freie Querschnitt des Kanals oder der Leitung an wenigstens einer Stelle begrenzt wird. Ein solches Ventil ist beispielsweise als Blende ausgebildet, die vorzugsweise in unterschiedliche Positionen und/oder Orientierungen relativ zu dem Kanal oder der Leitung gebracht, bevorzugt gedreht werden kann, wodurch sich der durchströmbare Querschnitt verändert, wodurch der Strömungswiderstand angepasst werden kann.

Vorzugsweise weist der Prothesenfuß ein Hydrauliksystem mit einer ersten Hydraulikkammer und einer zweiten Hydraulikkammer auf, die durch wenigstens eine Hydraulikleitung verbunden sind, wobei zum Verändern der Orientierung Hydraulikfluid von einer Hydraulikkammer in die andere Hydraulikkammer geleitet wird, wobei das Hydrauliksystem eine Ventilanordnung aufweist, durch die die Hydraulikleitung verschlossen ist, wenn sich die Sperreinrichtung in der Sperrstellung befindet. Vorzugsweise ist die Hydraulikleitung geöffnet, wenn sich die Sperreinrichtung in der Freigabestellung befindet.

**In** einer bevorzugten Ausgestaltung weist die Ventilanordnung ein Betätigungselement auf, das in eine Durchflussstellung und in eine Schließstellung bringbar ist und derart eingerichtet und ausgebildet ist, dass es durch das Schaltmoment aus der Durchflussstellung in die Schließstellung gebracht wird.

Vorteilhafterweise erfolgt eine Bewegung des Betätigungselementes aus der Durchflussstellung in die Schließstellung gegen eine Dämpfungskraft, die vorzugsweise durch wenigstens ein Reibelement und/oder ein viskoses Element hervorgerufen wird. Das Betätigungselement ist beispielsweise ein Ventilkörper oder ein anderes Bauteil der Ventilanordnung, das aus der Durchflussstellung in die Schließstellung gebracht wird, indem es entlang eines vorbestimmten Pfades verschoben wird. **In** einer bevorzugten Ausgestaltung wird diese Bewegung durch wenigstens ein Reibelement, beispielsweise einem oder mehreren Dichtringen, gebremst, also gedämpft. Je stärker die Dämpfung ist, desto langsamer erfolgt die Bewegung des Betätigungselementes und desto länger dauert es, bis die Ventilanordnung die Hydraulikleitung verschließt und so die Sperreinrichtung in die Sperrstellung bringt. Die dem Träger des Prothesenfußes zur Verfügung stehende Zeitspanne zwischen dem Aufbringen des Schaltmomentes und dem Zeitpunkt, in dem die Sperreinrichtung in der Sperrstellung ist, lässt sich auf diese Weise einstellen. Alternativ oder zusätzlich dazu kann die Bewegung des Betätigungselementes von der Durchflussstellung in die Schließstellung auch innerhalb eines viskosen Mediums erfolgen. Je viskoser das Medium ist, desto zähflüssiger ist es und desto länger braucht das Betätigungselement, um von der Durchflussstellung in die Schließstellung zu gelangen.

**In** einer bevorzugten Ausgestaltung weist die Ventilanordnung ein Hydraulikvolumen auf, in dem sich das Betätigungselement befindet und das mittels einer ersten Hydraulikverbindung mit der ersten Hydraulikkammer und mittels einer zweiten Hydraulikverbindung mit der zweiten Hydraulikkammer verbunden ist, wobei die erste Hydraulikverbindung durch ein erstes Ventil und die zweite Hydraulikverbindung durch ein zweites Ventil verschließbar ist. Vorzugsweise sind das erste Ventil und das zweite Ventil durch das Betätigungselement geöffnet, wenn sich das Betätigungselement in der Durchflussstellung befindet. Das Betätigungselement ist in diesem Fall nicht der Ventilkörper oder ein anderes Bauteil, das die jeweilige Verbindung verschließt, sondern sorgt lediglich dafür, dass diese Bauteile oder Ventilkörper aus der Schließstellung in die Durchflussstellung gebracht werden, wenn das Betätigungselement betätigt wird.

Vorzugsweise werden das erste Ventil und das zweite Ventil gleichzeitig geschlossen, nachdem das Betätigungselement durch das Schaltmoment in die Schließstellung gebracht wurde. Dies ist insbesondere dann von Vorteil, wenn keine Bewegung zwischen dem Fußelement und dem proximalen Anschlusselement möglich ist, solange sich die Sperreinrichtung in der Sperrstellung befindet.

**In** einer alternativen Ausgestaltung werden das erste Ventil und das zweite Ventil nacheinander geschlossen, nachdem das Betätigungselement in die Schließstellung gebracht wurde. Dies ist insbesondere dann von Vorteil, wenn eine Bewegung zwischen dem Fußelement und dem Anschlusselement auch dann möglich ist, wenn sich die Sperreinrichtung in der Sperrstellung befindet. Die Orientierung zwischen dem Fußelement und dem Anschlusselement kann jedoch auch in dieser Ausführungsform nur dann geändert werden, wenn sich die Sperreinrichtung in der Freigabestellung befinden.

Die Erfindung löst die gestellte Aufgabe zudem durch einen Prothesenfuß mit einem Fußelement, einem proximalen Anschlusselement, das an dem Fußelement angeordnet ist, und einer Sperreinrichtung, die in eine Freigabestellung und in einer Sperrstellung bringbar ist, wobei die Orientierung, in der das Anschlusselement an dem Fußelement angeordnet ist, veränderbar ist, wenn sich die Sperreinrichtung in der Freigabestellung befindet, wobei die Sperreinrichtung ein erstes Formschlusselement und ein zweites Formschlusselement aufweist, die miteinander in Eingriff sind, wenn sich die Sperreinrichtung in der Sperrstellung befindet und die außer Eingriff sind, wenn sich die Sperreinrichtung in der Freigabestellung befindet.

Vorzugsweise ist das erste Formschlusselement Teil des Fußelementes und das zweite Formschlusselement Teil des proximalen Anschlusselementes. **In** einer bevorzugten Ausgestaltung werden die beiden Formschlusselemente außer Eingriff gebracht, indem das Fußelement relativ zudem Anschlusselement um eine Schaltachse, die vorzugsweise eine vertikale Achse ist oder zumindest von proximal nach distal verläuft, verschwenkt werden. Besonders bevorzugt verfügt die Sperreinrichtung über ein Sicherungselement, das aus seiner Sicherungsposition entfernt werden muss, um die beiden Formschlusselemente außer Eingriff zu bringen.

Zum Einstellen einer Absatzhöhe in diesem Ausführungsbeispiel wird also zunächst das Sicherungselement aus seiner Sicherungsposition entfernt. **In** einem zweiten Schritt werden die beiden Formschlusselemente außer Eingriff gebracht. Dies kann beispielsweise geschehen, in dem die beiden Bauteile, an denen die jeweiligen Formschlusselemente angeordnet sind, relativ zueinander bewegt werden. Dies kann beispielsweise das Fußelement und das proximale Anschlusselement sein, die beispielsweise um eine Schwenkachse gegeneinander verschwenkt werden. Die Bewegung kann durchaus klein sein, sofern sie ausreichend ist, um die Formschlusselemente außer Eingriff zu bringen. Anschließend kann der beabsichtigte Plantarflexionswinkel eingestellt werden, indem beispielsweise ein Drehmoment um die einzustellende Achse aufgebracht wird. Dadurch kann das Fußelement relativ zum proximalen Anschlusselement verkippt oder verdreht werden und der gewünschte Winkel eingestellt werden. Anschließend wird die zum außer Eingriff bringen vorgenommene Bewegung rückgängig gemacht. Auf diese Weise werden die beiden Formschlusselemente wieder in Eingriff gebracht. Danach ist eine Veränderung des Plantarflexionswinkels nicht möglich, ohne die beiden Formschlusselemente wieder außer Eingriff zu bringen. Schließlich wird das Sicherungselement wieder in seine Sicherungsposition gebracht, um ein versehentliches außer Eingriff Bringen zu verhindern.

Mithilfe der beigefügen Figuren werden nachfolgend einigen Ausführungsformen der Erfindung näher erläutert. Es zeigen:
- Figur 1 und 2-: schematische Darstellungen eines Teils eines Prothesenfußes gemäß einem ersten Ausführungsbeispiel der vorliegenden Erfindung,
- Figuren 3 bis 5-: schematische Darstellungen eines Teil eines Prothesenfußes gemäß einem anderen Ausführungsbeispiel der vorliegenden Erfindung,
- Figur 6 -: die schematische Skizze der Funktion einer weiteren Ausgestaltung,
- Figuren 7 bis 10-: schematische Darstellungen einer anderen Ausgestaltung eines Prothesenfußes
- Figur 11 -: die schematische Schnittdarstellung durch einen Ausschnitt eines Prothesenfußes und
- Figur 12 -: die schematische Darstellung eines Teils aus Figur 11 in einer anderen Ansicht.

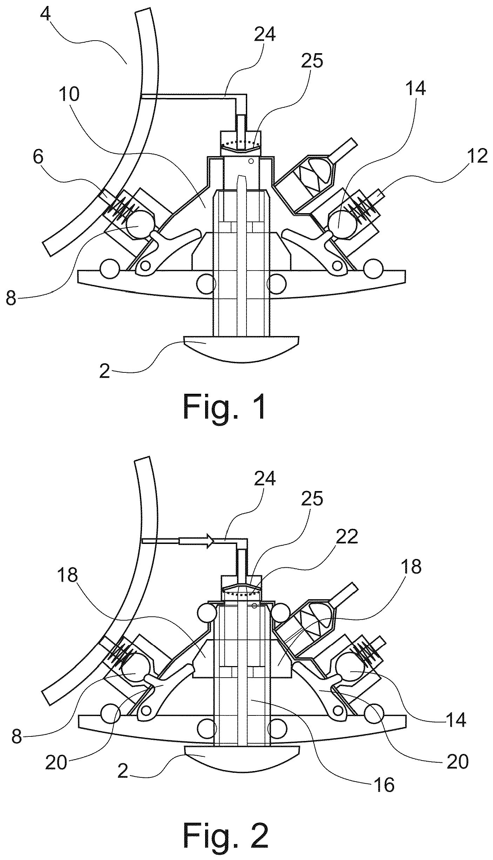

Figur 1 zeigt einen Teil eines Hydrauliksystems und eine Sperreinrichtung des Prothesenfußes. Diese verfügt über ein Betätigungselement 2 in Form eines Druckknopfes. Das Hydrauliksystem verfügt über eine erste Hydraulikkammer und eine zweite Hydraulikkammer, die miteinander verbunden sind, sodass Hydraulikfluid beim Verändern der Orientierung des Fußelementes relativ zum proximalen Anschlusselement von der einen Hydraulikkammer in die andere Hydraulikkammer geleitet wird. Die erste Hydraulikkammer 4 ist in Figur 1 abschnittsweise zu erkennen.

Das Betätigungselement 2 befindet sich wie die Sperreinrichtung in der Sperrstellung. Die erste Hydraulikkammer 4 ist über eine erste Hydraulikverbindung 6, in der sich ein erstes Rückschlagventil 8 befindet, mit einem Hydraulikvolumen 10 verbunden, in das das Betätigungselement 2 hineinragt. **In** der gezeigten Darstellung ist das erste Rückschlagventil 8 geschlossen, sodass kein Fluid aus der ersten Hydraulikkammer 4 in das Hydraulikvolumen 10 strömen kann. Die nicht dargestellte zweite Hydraulikkammer ist über eine zweite Hydraulikverbindung 12, in der sich ein zweites Hydraulikventil 14 befindet, mit dem Hydraulikvolumen 10 verbunden.

Figur 2 zeigt die Ausgestaltung aus Figur 1, wobei das Betätigungselement 2 betätigt wurde, in dem es in der gezeigten Darstellung nach oben gedrückt wurde. Das Betätigungselement 2 verfügt über einen Kolben 16, der in das Hydraulikvolumen 10 hineinragt und der über Vorsprünge 18 verfügt. Wird das Betätigungselement 2 wie in Figur 2 dargestellt betätigt, verschiebt sich auch der Kolben 16 und damit die Vorsprünge 18. Die Vorsprünge 18 öffnen das erste Rückschlagventil 8 und das zweite Rückschlagventil 14, indem sie die Schalthebel 20 bewegen. Diese drücken die Kugeln der Rückschlagventile 8, 14 in der gezeigten Darstellung nach oben und öffnen so die erste Fluidverbindung 6 und die zweite Fluidverbindung 12. Damit kann Fluid aus den beiden Hydraulikkammern in das Fluidvolumen 10 strömen.

Der Kolben 16 verfügt an seinem dem Betätigungselement 2 abgewandten Ende über einen Stößel 22, der beim Betätigen des Betätigungselementes 2 ebenfalls verschoben wird. Zwischen dem Hydraulikvolumen 10 und der ersten Hydraulikkammer 4 existiert eine Schaltleitung 24, die es jedoch nicht erlaubt, dass Fluid zwischen der ersten Hydraulikkammer 4 und dem Hydraulikvolumen 10 ausgetauscht wird. Die erste Hydraulikkammer 4 ist von dem Hydraulikvolumen 10 durch eine Membran 25 getrennt, die zwei stabile Zustände hat. Sie kann auch als bistabile Membran 25 bezeichnet werden. Die Membran 25 ist beispielsweise aus einem Metall hergestellt. In der gezeigten Ausführungsform ist die Membran 25 in einem ersten stabilen Zustand, der in Figur 1 dargestellt ist, nach unten gewölbt. Wird nun das Betätigungselement 2 betätigt und der Kolben 16 mit dem Stößel 22 bewegt, drückt der Stößel 22 gegen die Membran 25 und bringt diese so aus dem in Figur 1 gezeigten ersten stabilen Zustand in den in Figur 2 gezeigt zweiten stabilen Zustand.

In dem in Figur 2 gezeigten Zustand des Betätigungselementes 2 befindet sich das Betätigungselement 2 in seiner Durchflussstellung, sodass Fluid zwischen den beiden Hydraulikkammern ausgetauscht werden kann. Um nun die Sperreinrichtung, zu der das Betätigungselement 2 in dieser Ausgestaltung gehört, wieder in die Sperrstellung zu bringen, muss ein Schaltmoment auf das nicht dargestellte Fußelement ausgeübt werden. Im gezeigten Ausführungsbeispiel ist dies erreichbar, indem der Vorfuß des Prothesenfußes belastet wird. Dies führt zu einer Erhöhung des Druckes in der ersten Hydraulikkammer 4 und damit auch in der mit dieser verbundenen Schaltleitung 24. Dadurch wird die Membran 25 wieder in den in Figur 1 gezeigten ersten stabilen Zustand gedrückt und das Betätigungselement 2 aus seiner Durchflussstellung, die in Figur 2 gezeigt ist, in seine Schließstellung gebracht. Dabei wird der Kolben 16 mit den Vorsprüngen 18 nach unten bewegt, wodurch auch die Schalthebel 20 sich zurück bewegen und die Rückschlagventile 8, 14 schließen. Die Sperreinrichtung befindet sich dann wieder in der Sperrstellung.

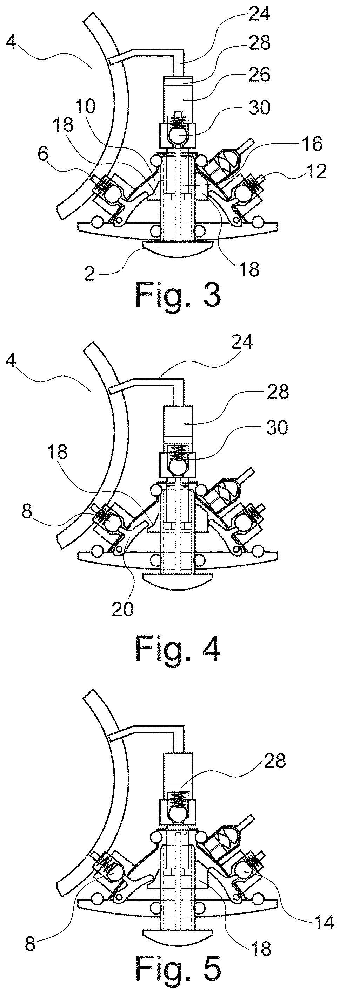

Die Figuren 3 bis 5 zeigen eine etwas andere Ausgestaltung, die der in den Figuren 1 und 2 gezeigten Ausgestaltung jedoch sehr ähnlich ist. Auch hier befindet sich das Hydraulikvolumen 10 zwischen den beiden Hydraulikkammern, von denen wieder nur die erste Hydraulikkammer 4 dargestellt ist. Dieser ist mit dem Hydraulikvolumen 10 über die erste Hydraulikverbindung 6 verbunden, in der sich das erste Rückschlagventil 8 befindet. Die zweite Hydraulikkammer, die in den Figuren 3 bis 5 ebenfalls nicht dargestellt ist, ist über die zweite Hydraulikverbindung 12 mit dem Hydraulikvolumen 10 verbunden. **In** der zweiten Hydraulikverbindung 12 befindet sich das zweite Rückschlagventil 14. **In** Figur 3 ist das Betätigungselement 2 betätigt worden und der Kolben 16 mit den Vorsprüngen 18 ist verschoben worden. Anders als in der Ausgestaltung der Figuren 1 und 2 sind die beiden Vorsprünge 18 in den Figuren 3 bis 5 nicht identisch ausgebildet. **In** der in Figur 3 gezeigten Situation sind jedoch beide Rückschlagventile 8, 14 geöffnet.

Ein weiterer Unterschied zwischen der Ausgestaltung der Figuren 3 bis 5 und der Ausgestaltung der Figuren 1 und 2 liegt in der Schaltleitung 24, der sich in den Figuren 3 bis 5 ein Schaltvolumen 26 mit einem fliegenden Kolben 28 befindet. Der fliegende Kolben 28 trennt das Schaltvolumen 26 in zwei Abschnitte, die auch als Kammern bezeichnet werden können. Der erste Abschnitt oberhalb des fliegenden Kolben 28 und ist über die Schaltleitung 24 mit der ersten Hydraulikkammer 4 verbunden. Der zweite Abschnitt unterhalb des fliegenden Kolben 28 ist durch ein weiteres Rückschlagventil 30 mit dem Hydraulikvolumen 10 verbunden.

**In** Figur 3 ist der Kolben 16 mit den Vorsprüngen 18 soweit nach oben verschoben, dass beide Schalthebel 20 die jeweiligen Rückschlagventile 8, 14 öffnen und somit eine Verbindung zwischen den beiden Hydraulikkammern und dem Hydraulikvolumen 10 ermöglichen. Wird in dieser Situation das Schaltmoment aufgebracht, das beispielsweise in einer Belastung des Vorfußes bestehen kann, wird dadurch der Druck innerhalb der ersten Hydraulikkammer 4 und damit auch in der Schaltleitung 24 erhöht. Der fliegende Kolben 28 wird nach unten bewegt, bis er das Schaltventil 30 schließt, wodurch das Betätigungselement 2 aus seiner Durchflussstellung in die Sperrstellung gebracht wird. Diese Situation ist in Figur 4 dargestellt.

Der in Figur 4 linke Vorsprung 18 des Kolbens 16 ist so ausgebildet, dass der entsprechende Schalthebel 20 sich ab der in Figur 4 gezeigten Position des sich nach unten bewegenden Kolbens 16 wieder bewegen kann und so aufgrund der herrschenden Drücke das erste Rückschlagventil 8 schließt. Diese Situation ist in Figur 5 dargestellt. Das erste Rückschlagventil 8 ist bereits geschlossen, während das zweite Rückschlagventil 14 aufgrund des größeren Vorsprung 18 noch geöffnet ist. Es ist daher weiterhin möglich, das Fluid aus der nicht gezeigten zweiten Hydraulikkammer durch das geöffnete zweite Rückschlagventil 14 in das Hydraulikvolumen 10 und von dort in den zweiten Bestandteil des Schaltvolumens 26 eindringt, wodurch der fliegende Kolben 28 wieder nach oben bewegt wird.

Dies bedeutet, dass selbst nach dem Schließen des ersten Rückschlagventil 8 eine Bewegung des Fußelementes relativ zum Anschlusselement möglich ist, indem der fliegende Kolben 28 bewegt wird. Damit lässt sich eine absatzhöhenunabhängige Positionierung der beiden Elemente zueinander erreichen.

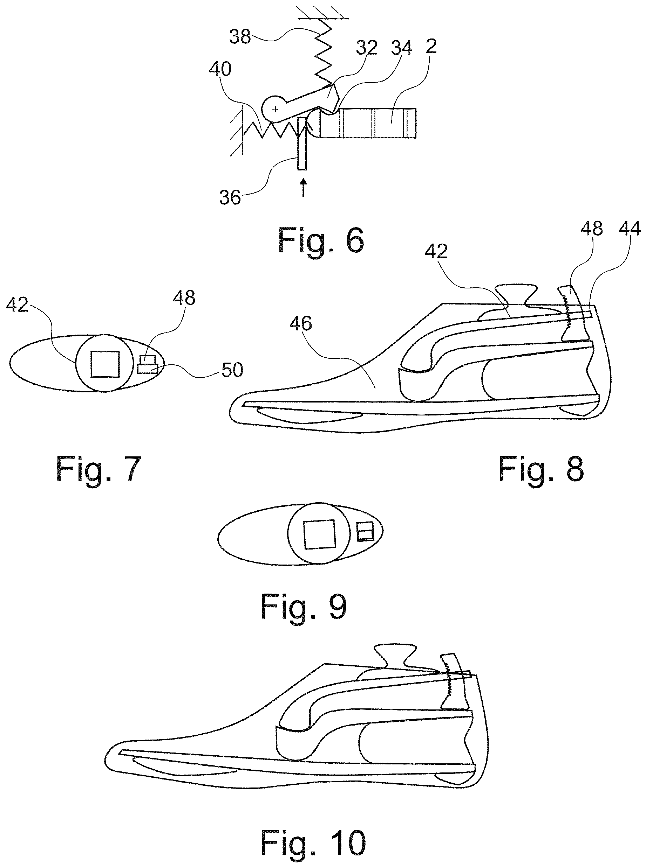

Figur 6 zeigt schematisch eine andere Ausgestaltung einer Sperreinrichtung. Man erkennt schematisch das Betätigungselement 2, das sich in Figur 6 in seiner Durchflussstellung befindet. Dazu wurde es beispielsweise in Figur 6 aus seiner Schließstellung von rechts nach links verschoben. Es hat die Durchflussstellung erreicht, wenn der Haken 32 in die dafür vorgesehene Ausnehmung 34 eingreift und das Betätigungselement 2 so in seiner Durchflussstellung hält. Durch das Aufbringen eines Schaltmomentes wird ein Stift 36 nach oben bewegt und sorgt so dafür, dass der Haken 32 nicht mehr in die Ausnehmung 34 eingreift, wobei die Haltefeder 38 komprimiert wird. Sobald der Haken 32 das Betätigungselement 2 nicht mehr festhält, wird dieses durch die Schubfeder 40 zurück in die Schließstellung gebracht.

Die Figuren 7 bis 10 zeigen eine andere Ausgestaltung eines Prothesenfußes. Figur 8 zeigt schematisch ein proximales Anschlusselement 42, an dem ein erstes Formschlusselement 44 angeordnet ist und das mit einem Fußelement 46 verbunden ist. An dem Fußelement 46 ist ein zweites Formschlusselement 48 angeordnet, das in der in Figur 8 gezeigten Situation mit dem ersten Formschlusselement 44 in Eingriff steht. Figur 7 zeigt eine Draufsicht, in der man einen Teil des Anschlusselementes 42 und des zweiten Formschlusselementes 48 erkennt. Zusätzlich ist ein Sicherungselement 50 dargestellt, durch das verhindert wird, dass das erste Formschlusselement 44 und das zweite Formschlusselement 48 außer Eingriff gebracht werden.

In Figur 9 ist das Sicherungselement 50 entfernt und das Fußelement 46 relativ zum Anschlusselement 42 um eine Schaltachse gedreht worden, die sich senkrecht zur Zeichenebene erstreckt. Man erkennt, dass das zweite Formschlusselement 48 anders als in Figur 7 nicht mehr am oberen Ende der Ausnehmung im Anschlusselement 42, sondern am unteren Ende dieser Ausnehmung positioniert ist. Dadurch wird dargestellt, dass das Fußelement 46 und damit auch das zweite Formschlusselement 48 relativ zum Anschlusselement 42 verdreht wurden. Dadurch sind das erste Formschlusselement 44 und das zweite Formschlusselement 48 außer Eingriff gebracht worden und das Fußelement 46 kann relativ zum Anschlusselement 42 in seiner Orientierung verändert werden. Dazu wird es, wie in Figur 10 dargestellt, um eine von medial nach lateral verlaufende Schwenkachse verdreht.

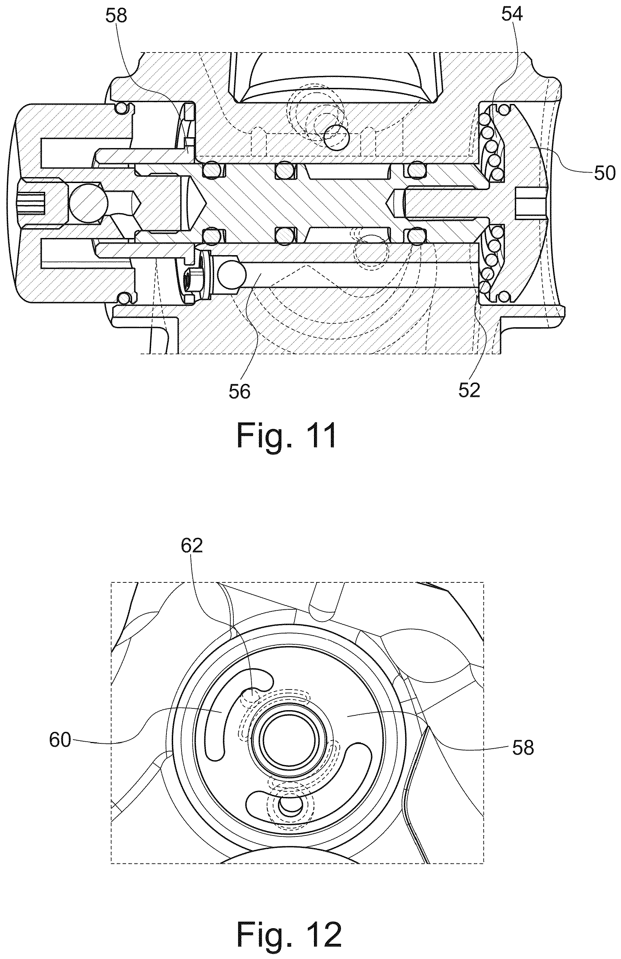

Figur 11 zeigt einen Ausschnitt einer schematischen Schnittdarstellung durch einen Teil einer Sperreinrichtung. Ein Bauteil 50, das im gezeigten Ausführungsbeispiel das Betätigungselement ist, mit dem die Sperreinrichtung aus der Sperrstellung in die Freigabestellung bringbar ist, befindet sich in der zweiten Position. Die Sperreinrichtung befindet sich daher in der Freigabestellung. Das Bauteil 50 wurde durch das Schaltmoment in die gezeigte Position gebracht worden. Durch die Feder 52 ist das Bauteil 50 einer Kraft ausgesetzt, die das Bauteil 50 im gezeigten Beispiel nach rechts in die erste Position verschiebt. Sobald das Bauteil 50 diese erste Position erreicht, befindet sich die Sperreinrichtung in der Sperrstellung.

Die Feder 52 befindet sich in einem ersten Volumen 54, das sich vergrößert, wenn sich die Feder 52 entspannt und das Bauteil 50 nach rechts bewegt. Daher wird ein Fluid, beispielsweise Luft durch eine nicht dargestellt Leitung in das Volumen 54 hineingesaugt. Durch die Leitung 56 ist das Fluid aus dem Volumen 54 herausgedrückt wurden, als sich das Bauteil 50 durch das Schaltmoment nach links bewegt und das Volumen 54 verkleinert hat. Der Strömungswiderstand, der der Rückströmung des Fluids zurück in das Volumen 54 entgegengesetzt wird, wird durch eine Blende 58, die die Funktion eines Drosselventils übernimmt, begrenzt. Dadurch kommt es zu einer Begrenzung der Geschwindigkeit, mit der die Feder 52 das Bauteil 50 bewegt, das daher seine Endposition verzögert erreicht.

Figur 12 zeigt die Blende 58 in einer Draufsicht. Sie weist ein Langloch 60 auf, das eine Öffnung 62 der Rückleitung, die in Figur 11 nicht gezeigt ist und durch die das Fluid in das Volumen 54 zurückströmt je nach Stellung der Blende 58 mehr oder weniger verschließt und so mehr oder weniger Strömungswiderstand aufbringt.

### Bezugszeichenliste

2 Betätigungselement
4 erste Hydraulikkammer
6 erste Hydraulikverbindung
8 erstes Rückschlagventil
10 Hydraulikvolumen
12 zweite Hydraulikverbindung
14 zweites Rückschlagventil
16 Kolben
18 Vorsprung
20 Schalthebel
22 Stößel
24 Schaltleitung
25 Membran
26 Schaltvolumen
28 fliegender Kolben
30 Rückschlagventil
32 Haken
34 Ausnehmung
36 Stift
38 Haltefeder
40 Schubfeder
42 Anschlusselement
44 erstes Formschlusselement
46 Fußelement
48 zweites Formschlusselement
50 Bauteil
52 Feder
54 Volumen
56 Leitung
58 Blende

## Patentansprüche

1. Prothesenfuß mit einem Fußelement, einem proximalen Anschlusselement (42) Ansehen das
an dem Fußelement angeordnet ist, und einer Sperreinrichtung, die in eine Freigabestellung und in eine Sperrstellung bringbar ist, wobei eine Orientierung, in der das Anschlusselement (42) an dem Fußelement angeordnet ist, veränderbar ist, wenn sich die Sperreinrichtung in der Freigabestellung befindet, **dadurch gekennzeichnet, dass**
die Sperreinrichtung derart ausgebildet ist, dass die Sperreinrichtung selbsttätig aus der Freigabestellung in die Sperrstellung gelangt, nachdem ein Schaltmoment auf das Fußelement aufgebracht wird.

2. Prothesenfuß nach Anspruch 1, **dadurch gekennzeichnet, dass** die Sperreinrichtung durch Betätigen eines mechanischen Betätigungselementes (2) aus der Sperrstellung in die Freigabestellung bringbar ist, wobei das Betätigungselement vorzugsweise durch das Aufbringen des Schaltmomentes betätigt wird.

3. Prothesenfuß nach Anspruch 1 oder 2, **dadurch gekennzeichnet, dass** der Prothesenfuß, vorzugsweise die Sperreinrichtung, ein Verzögerungselement aufweist, das ausgebildet und eingerichtet ist, die Wirkung des Schaltmomentes, die Sperreinrichtung aus der Freigabestellung in die Sperrstellung zu bringen, zu verzögern.

4. Prothesenfuß nach einem der vorstehenden Ansprüche, **dadurch gekennzeichnet, dass** das Fußelement um eine Schwenkachse relativ zu dem Anschlusselement schwenkbar ist und das Schaltmoment ein Drehmoment um die Schwenkachse ist.

5. Prothesenfuß nach Anspruch 4, **dadurch gekennzeichnet, dass** sich die Schwenkachse von einem Fersenbereich des Prothesenfußes zu einem Vorfußbereich des Prothesenfußes erstreckt.

6. Prothesenfuß nach einem der vorstehenden Ansprüche, **dadurch gekennzeichnet, dass** der Prothesenfuß ein Hydrauliksystem mit einer ersten Hydraulikkammer (4) und einer zweiten Hydraulikkammer aufweist, die durch wenigstens eine Hydraulikleitung verbunden sind, wobei zum Verändern der Orientierung Hydraulikfluid von einer Hydraulikkammer in die andere Hydraulikkammer geleitet wird, wobei das Hydrauliksystem eine Ventilanordnung aufweist, durch die die Hydraulikleitung verschlossen ist, wenn sich die Sperreinrichtung in der Sperrstellung befindet.

7. Prothesenfuß nach Anspruch 6, **dadurch gekennzeichnet, dass** die Ventilanordnung ein Betätigungselement aufweist, das in eine Durchflussstellung und eine Schließstellung bringbar ist, und derart ausgebildet und eingerichtet ist, dass es durch das Schaltmoment aus der Durchflussstellung in die Schließstellung gebracht wird.

8. Prothesenfuß nach Anspruch 7, **dadurch gekennzeichnet, dass** eine Bewegung des Betätigungselements (2) aus der Durchflussstellung in die Schließstellung gegen eine Dämpfungskraft erfolgt, die vorzugsweise durch wenigstens ein Reibelement und/oder ein viskoses Element hervorgerufen wird.

9. Prothesenfuß nach Anspruch 7 oder 8, **dadurch gekennzeichnet, dass** die Ventilanordnung ein Hydraulikvolumen (10) aufweist, in dem sich das Betätigungselement (2) befindet und das mittels einer ersten Hydraulikverbindung (6) mit der ersten Hydraulikkammer und mittels einer zweiten Hydraulikverbindung (12) mit der zweiten Hydraulikkammer verbunden ist, wobei die erste Hydraulikverbindung durch ein erstes Ventil und die zweite Hydraulikverbindung (12) durch ein zweites Ventil verschließbar ist.

10. Prothesenfuß nach Anspruch 9, **dadurch gekennzeichnet, dass** das erste Ventil und das zweite Ventil durch das Betätigungselement (2) geöffnet sind, wenn sich das Betätigungselement in der Durchflussstellung befindet.

11. Prothesenfuß nach Anspruch 10, **dadurch gekennzeichnet, dass** das erste Ventil und das zweite Ventil gleichzeitig geschlossen werden, nachdem das Betätigungselement (2) in die Schließstellung gebracht wurde.

12. Prothesenfuß nach Anspruch 10, **dadurch gekennzeichnet, dass** das erste Ventil und das zweite Ventil nacheinander geschlossen werden, nachdem das Betätigungselement (2) in die Schließstellung gebracht wurde.

## Claims

1. A prosthetic foot with a foot element, a proximal connection element (42) arranged on the foot element and a blocking device that can be brought into a release position and a blocking position, wherein an orientation in which the connection element (42) is arranged on the foot element can be modified when the blocking device is in the release position,
**characterized in that**
the blocking device is designed in such a way that the blocking device automatically moves from the release position into the blocking position once a switching torque is applied to the foot element.

2. The prosthetic foot according to claim 1, **characterized in that** the blocking device can be brought out of the blocking position into the release position by actuating a mechanical actuation element (2), wherein the actuation element is preferably actuated by applying the switching torque.

3. The prosthetic foot according to claim 1 or 2, **characterized in that** the prosthetic foot, preferably the blocking device, comprises a delay element that is designed and configured to delay the effect of the switching torque of moving the blocking device out of the release position into the blocking position.

4. The prosthetic foot according to one of the preceding claims, **characterized in that** the foot element can be swivelled relative to the connection element about a swivel axis and the switching torque is a torque about the swivel axis.

5. The prosthetic foot according to claim 4, **characterized in that** the swivel axis extends from a heel area of the prosthetic foot to a forefoot area of the prosthetic foot.

6. The prosthetic foot according to one of the preceding claims, **characterized in that** the prosthetic foot comprises a hydraulic system with a first hydraulic chamber (4) and a second hydraulic chamber that are connected by at least one hydraulic line, wherein hydraulic fluid is directed from one hydraulic chamber into the other hydraulic chamber in order to modify the orientation, wherein the hydraulic system comprises a valve arrangement by means of which the hydraulic line is closed when the blocking device is in the blocking position.

7. The prosthetic foot according to claim 6, **characterized in that** the valve arrangement comprises an actuation element which can be brought into a through-flow position and a closing position, and which is configured and designed in such a way that it is brought out of the through-flow position into the closing position by the switching torque.

8. The prosthetic foot according to claim 7, **characterized in that** the actuation element (2) moves out of the through-flow position into the closing position against a damping force, which is preferably generated by at least one friction element and/or one viscous element.

9. The prosthetic foot according to claim 7 or 8, **characterized in that** the valve arrangement comprises a hydraulic volume (10) in which the actuation element (2) is located and which is connected to the first hydraulic chamber by means of a first hydraulic connection (6) and to the second hydraulic chamber by means of a second hydraulic connection (12), wherein the first hydraulic connection can be closed by a first valve and the second hydraulic connection (12) by a second valve.

10. The prosthetic foot according to claim 9, **characterized in that** the first valve and the second valve are opened by the actuation element (2) when the actuation element is in the through-flow position.

11. The prosthetic foot according to claim 10, **characterized in that** the first valve and the second valve are closed at the same time once the actuation element (2) has been brought into the closing position.

12. The prosthetic foot according to claim 10, **characterized in that** the first valve and the second valve are closed one after the other once the actuation element (2) has been brought into the closing position.

## Revendications

1. Prothèse de pied comprenant un élément de pied, un élément de raccordement proximal (42) disposé sur l'élément de pied, et un dispositif de verrouillage qui peut être amené dans une position de déverrouillage et dans une position de verrouillage, l'orientation selon laquelle l'élément de raccordement (42) est disposé sur l'élément de pied pouvant être modifiée lorsque le dispositif de verrouillage se trouve dans la position de déverrouillage,
**caractérisée en ce que** le dispositif de verrouillage est conçu de telle sorte que le dispositif de verrouillage passe automatiquement de la position de déverrouillage à la position de verrouillage après qu'un couple de commutation a été appliqué à l'élément de pied.

2. Prothèse de pied selon la revendication 1,
**caractérisée en ce que** le dispositif de verrouillage peut être amené de la position de verrouillage à la position de déverrouillage par l'actionnement d'un élément d'actionnement mécanique (2), l'élément d'actionnement étant actionné de préférence par l'application du couple de commutation.

3. Prothèse de pied selon la revendication 1 ou 2,
**caractérisée en ce que** la prothèse de pied, de préférence le dispositif de verrouillage, comporte un élément de retardement qui est conçu et agencé pour retarder l'effet du couple de commutation visant à faire passer le dispositif de verrouillage de la position de déverrouillage à la position de verrouillage.

4. Prothèse de pied selon l'une des revendications précédentes,
**caractérisée en ce que** l'élément de pied peut pivoter autour d'un axe de pivotement par rapport à l'élément de raccordement, et **en ce que** le couple de commutation est un couple de rotation autour de l'axe de pivotement.

5. Prothèse de pied selon la revendication 4,
**caractérisée en ce que** l'axe de pivotement s'étend d'une zone du talon de la prothèse de pied jusqu'à une zone de l'avant-pied de la prothèse de pied.

6. Prothèse de pied selon l'une des revendications précédentes, **caractérisée en ce que** la prothèse de pied comporte un système hydraulique comprenant une première chambre hydraulique (4) et une deuxième chambre hydraulique, qui sont reliées par au moins une conduite hydraulique, sachant que pour modifier l'orientation, un fluide hydraulique est acheminé d'une chambre hydraulique vers l'autre chambre hydraulique, le système hydraulique comportant un dispositif à clapet par lequel la conduite hydraulique est fermée lorsque le dispositif de verrouillage se trouve en position de verrouillage.

7. Prothèse de pied selon la revendication 6,
**caractérisée en ce que** le dispositif à clapet comprend un élément d'actionnement qui peut être amené dans une position d'écoulement et dans une position de fermeture et qui est conçu et agencé de telle sorte qu'il est amené de la position d'écoulement à la position de fermeture par le couple de commutation.

8. Prothèse de pied selon la revendication 7,
**caractérisée en ce que** le déplacement de l'élément d'actionnement (2) de la position d'écoulement vers la position de fermeture s'effectue à l'encontre d'une force d'amortissement qui est de préférence provoquée par au moins un élément de frottement et/ou un élément visqueux.

9. Prothèse de pied selon la revendication 7 ou 8,
**caractérisée en ce que** le dispositif à clapet présente un volume hydraulique (10) dans lequel se trouve l'élément d'actionnement (2) et qui est relié à la première chambre hydraulique au moyen d'une première communication hydraulique (6) et à la deuxième chambre hydraulique au moyen d'une deuxième communication hydraulique (12), la première communication hydraulique pouvant être fermée par un premier clapet et la deuxième communication hydraulique (12) pouvant être fermée par un deuxième clapet.

10. Prothèse de pied selon la revendication 9,
**caractérisée en ce que** le premier clapet et le deuxième clapet sont ouverts par l'élément d'actionnement (2) lorsque l'élément d'actionnement se trouve en position d'écoulement.

11. Prothèse de pied selon la revendication 10,
**caractérisée en ce que** le premier clapet et le deuxième clapet sont fermés simultanément après que l'élément d'actionnement (2) a été amené dans la position de fermeture.

12. Prothèse de pied selon la revendication 10,
**caractérisée en ce que** le premier clapet et le deuxième clapet sont fermés successivement après que l'élément d'actionnement (2) a été amené dans la position de fermeture.
